# EUROPEAN PATENT APPLICATION

(11) **EP 2 891 493 A1**
(43) Date of publication of application: **08.07.2015**
(21) Application number: 13832156.7
(22) Date of filing: 30.08.2013
(51) Int. Cl.: A61K 31/4725, A61K 9/24, A61K 31/426, A61K 47/12, A61K 47/32, A61K 47/34, A61K 47/38, A61P 13/00

(54) **ORALLY ADMINISTERED MEDICAL COMPOSITION**

(30) Priority: 31.08.2012 JP 2012191833
(71) Applicant: Astellas Pharma Inc., Chuo-ku Tokyo 103-8411 (JP)
(72) Inventor: TSUTSUI, Yuuki, Tokyo 103-8411 (JP); TOYOTA, Hiroyasu, Tokyo 103-8411 (JP); HAKOMORI, Tadashi, Tokyo 103-8411 (JP)
(74) Representative: Hoffmann Eitle
(86) International application number: PCT/JP2013/073351
(87) International publication number: WO 2014/034860

(57) **Abstract**

In order to provide the medical field with a single formulation comprising a modified release portion containing mirabegron or a pharmaceutically acceptable salt thereof and an immediate release portion containing solifenacin or a pharmaceutically acceptable salt thereof, (1) a single formulation having dissolution rates of both drugs similar to those of the current single drug formulations is provided, and (2) a single formulation having maximum percentages of dissolution of both drugs of 90% or more, and having a bioavailability equivalent to those of the current single drug formulations. Further, in order to provide a single formulation, (3) a single formulation having good productivity whereby failures in tabletting are reduced, and having good storage stability whereby the coloration of the immediate release portion is suppressed is provided. The pharmaceutical composition for oral administration of the present invention contains (1) a modified release portion comprising mirabegron or a pharmaceutically acceptable salt thereof, and (2) an immediate release portion comprising solifenacin or a pharmaceutically acceptable salt thereof, and calcium stearate.

## Description

### TECHNICAL FIELD

The present invention relates to a pharmaceutical composition for oral administration comprising a modified release portion capable of controlling the release of mirabegron, and an immediate release portion capable of rapidly releasing solifenacin.

More specifically, the present invention relates to a pharmaceutical composition for oral administration comprising the modified release portion containing mirabegron or a pharmaceutically acceptable salt thereof, a hydrogel-forming polymer, and a hydrophilic base, and the immediate release portion containing solifenacin or a pharmaceutically acceptable salt thereof, and calcium stearate, in a single formulation.

### BACKGROUND ART

Mirabegron is also known as YM178, and is a compound having the following structural formula. Its chemical name is (R)-2-(2-aminothiazol-4-yl)-4'-{2-[(2-hydroxy-2-phenylethyl)amino]ethyl}acetanilide (also known as 2-(2-amino-1,3-thiazol-4-yl)-N-[4-(2-{[(2R)-2-hydroxy-2-phenylethyl]amino}ethyl)phenyl]acetamide). It is known that mirabegron or its pharmaceutically acceptable salts have a β3-adrenergic receptor agonist activity, and are useful as a therapeutic agent for overactive bladder (Patent literatures 1-3).

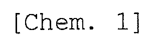

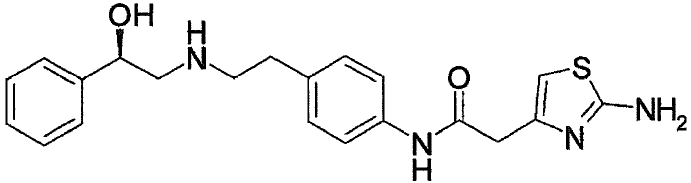

A tablet containing mirabegron is placed on the market, as a selective β3-adrenergic receptor-operated therapeutic agent for overactive bladder, as "Betanis (registered trademark) tablet" in Japan.

In clinical studies conducted in the development phase of the mirabegron, it is known that its pharmacokinetics vary depending on the presence or absence of food intake (Patent literature 4). When pharmacokinetics varies according to the presence or absence of food intake, it inevitably affects its functions and effects. Particularly, in medicaments, when functions and effects different from the predicted ones occur, it is considered that it may cause an unexpected situation, and thus, it is required that certain functions and effects can be predicted. Under circumstances where it is strongly desired to develop a drug with a minimum change in pharmacokinetics by the presence or absence of food intake, it is known that the change in pharmacokinetics of mirabegron by the presence or absence of food intake can be decreased by controlling the drug release using various additives (Patent literature 4).

Solifenacin is also known as YM905, and is a compound having the following structural formula. Its chemical name is (R)-quinuclidin-3-yl (S)-1-phenyl-1,2,3,4-tetrahydroisoquinoline-2-carboxylate (also known as (3R)-1-azabicyclo[2.2.2]oct-3-yl (1S)-1-phenyl-3,4-dihydroisoquinoline-2(1H)-carboxylate).

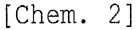

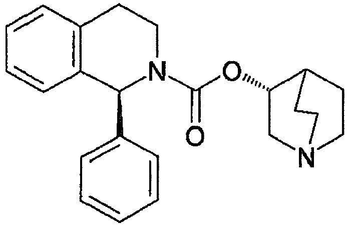

It is known that solifenacin or pharmaceutically acceptable salts thereof have a selective antagonistic activity against a muscarinic M₃ receptor, and are useful as an agent for preventing and/or treating various diseases (Patent literature 5).

A tablet containing solifenacin succinate is placed on the market, as a therapeutic agent for overactive bladder, as Vesicare (registered trademark) in Japan, VESIcare (registered trademark) in the United States, and Vesicare (registered trademark) in Europe.

In order to treat urinary urgency, urinary frequency, and/or urge urinary incontinence associated with overactive bladder, inventions relating to a pharmaceutical composition containing mirabegron or a pharmaceutically acceptable salt thereof, a pharmaceutical composition containing solifenacin or a pharmaceutically acceptable salt thereof, and a combination use of both drugs, are disclosed (Patent literature 6).

For the treatment of urinary urgency, urinary frequency, and/or urge urinary incontinence associated with overactive bladder, it is desired to provide the medical field with both the mirabegron or a pharmaceutically acceptable salt thereof and solifenacin or a pharmaceutically acceptable salt thereof, as a single formulation (i.e., a combined formulation), in order to improve drug dosing compliance. However, because the drug dissolution profiles in both formulations are different from each other, even when a single formulation (combined forumulation) is prepared from both formulations, it is desired to provide a single formulation in which the drug releasing profile in each portion contained in the single formulation is not much affected.

In connection with this, as a pharmaceutical composition for oral administration comprising solifenacin and other drug(s) (a single formulation, i.e., a combined formulation), a pharmaceutical composition for oral administration comprising a modified release portion containing tamsulosin or a pharmaceutically acceptable salt thereof, a hydrophilic base, and a hydrogel-forming polymer, and an immediate release portion containing solifenacin or a pharmaceutically acceptable salt thereof, and a hydrophilic substance, in a single formulation, is known (Patent literature 7).

### CITATION LIST

### PATENT LITERATURE

[Patent literature 1] WO 2004/041276
[Patent literature 2] WO 99/20607
[Patent literature 3] WO 03/037881
[Patent literature 4] WO 2010/038690
[Patent literature 5] US 6,017,927 (corresponding to WO 96/20194)
[Patent literature 6] WO 2009/057685
[Patent literature 7] WO 2010/090172

### SUMMARY OF INVENTION

### TECHNICAL PROBLEM

Objects of the present invention are, in providing the medical field with a single formulation (i.e., a combined formulation) comprising a modified release portion containing mirabegron or a pharmaceutically acceptable salt thereof and an immediate release portion containing solifenacin or a pharmaceutically acceptable salt thereof, (1) to provide a single formulation (a combined formulation) having dissolution profiles (drug dissolution rates in a predetermined period of time) of both drugs (in particular, solifenacin in the immediate release portion) similar to those of the current single drug formulations, and (2) to provide a single formulation (a combined formulation) having maximum percentages of dissolution of both drugs (in particular, solifenacin in the immediate release portion) of 90% or more, and having a bioavailability equivalent to those of the current single drug formulations. Further, in providing the single formulation (i.e., a combined formulation), it is (3) to provide a single formulation (a combined formulation) having good productivity whereby failures in tableting, such as lamination and sticking, are suppressed, and a good storage stability whereby coloration of the immediate release portion is suppressed.

### SOLUTION TO PROBLEM

The present inventors used the same components as those of a modified release pharmaceutical composition containing mirabegron, described in Example 10 of Patent literature 4, and an immediate release portion containing solifenacin succinate, described in Example 1 [(2) immediate release portion] of Patent literature 7, to prepare a single formulation (bi-layered tablets) (Comparative Example 1 described below). A dissolution test was carried out using the obtained bi-layered tablets to unexpectedly find:
[1] that the dissolution rate of solifenacin was less than 85% after 15 minutes, and
[2] that the maximum percentage of solifenacin dissolution was less than 90%.

When the dissolution rate or the maximum percentage of solifenacin dissolution is decreased, it is concerned that a decreased availability in the living body, i.e., bioavailability, will be caused and, as a result, pharmacological effects equivalent to those obtained by the combination use of the current formulations (single drug formulations) cannot be obtained. Further, in providing a single formulation (combined formulation), failures in tableting, such as lamination and sticking, and coloration of the immediate portion during storage, were observed.

The solubility of solifenacin in water is 610 mg/mL, and the solubility of solifenacin in the 2nd fluid (pH 6.8) for the Dissolution Test described in the Japanese Pharmacopoeia is 430 mg/mL, and thus, solifenacin is classified into "soluble" substances in a neutral solvent, such as water, in accordance with the expression of solubility described in the Japanese Pharmacopoeia. Further, the current product (Vesicare (registered trademark)) has been placed on the market, as a drug-immediate-release formulation. Furthermore, although the active ingredient is not the same, a bi-layered tablet comprising a modified release portion containing tamsulosin or a pharmaceutically acceptable salt thereof, and an immediate release portion containing solifenacin or a pharmaceutically acceptable salt thereof, is known (Patent literature 7). However, the present inventors found that, when mirabegron was used instead of tamsulosin hydrochloride, the release rate of solifenacin and the maximum percentage of solifenacin dissolution, from the bi-layered tablet (Comparative Example 1 of the present specification) prepared using the same components as those of the immediate release portion containing solifenacin succinate, described in Patent literature 7 [(2) immediate release portion in Example 1], were decreased in comparison with the single drug formulation.

Under these circumstances, the present inventors focused attention on the improvement of the release rate of solifenacin and the maximum percentage of solifenacin dissolution, conducted intensive studies, and completed the present invention.

The present invention provides:
[1] a pharmaceutical composition for oral administration comprising (1) a modified release portion comprising mirabegron or a pharmaceutically acceptable salt thereof, and (2) an immediate release portion comprising solifenacin or a pharmaceutically acceptable salt thereof, and calcium stearate,
[2] the pharmaceutical composition for oral administration of [1], wherein the immediate release portion is disintegrated and/or dissolved before the modified release portion forms a gel,
[3] the pharmaceutical composition for oral administration of [1] or [2], wherein about 85% or more of solifenacin is dissolved after 15 minutes,
[4] the pharmaceutical composition for oral administration of [3], wherein 90% or more of solifenacin is dissolved after 60 minutes,
[5] the pharmaceutical composition for oral administration of any one of [1] to [4], wherein the content of calcium stearate is about 0.1% by weight to about 10% by weight with respect to the weight of the immediate release portion,
[6] the pharmaceutical composition for oral administration of any one of [1] to [5], wherein the modified release portion contains a polymer which forms a hydrogel,
[7] the pharmaceutical composition for oral administration of [6], wherein the hydrogel-forming polymer has an average molecular weight of about 100,000 or more, or a viscosity of 12 mPa·s or more in a 5% aqueous solution at 25°C,
[8] the pharmaceutical composition for oral administration of [7], wherein the hydrogel-forming polymer is one polymer or two or more polymers selected from the group consisting of polyethylene oxide, hypromellose, hydroxypropylcellulose, methylcellulose, carboxymethylcellulose sodium, hydroxyethylcellulose, and a carboxyvinyl polymer,
[9] the pharmaceutical composition for oral administration of [8], wherein the hydrogel-forming polymer is polyethylene oxide,
[10] the pharmaceutical composition for oral administration of any one of [6] to [9], wherein the content of the hydrogel-forming polymer is about 1% by weight to about 70% by weight with respect to the weight of the modified release portion,
[11] the pharmaceutical composition for oral administration of any one of [1] to [10], wherein the modified release portion further contains an additive which allows water to penetrate into the modified release portion,
[12] the pharmaceutical composition for oral administration of [11], wherein the additive which allows water to penetrate into the modified release portion has a solubility such that the amount of water necessary to dissolve 1 g of the additive is 10 mL or less,
[13] the pharmaceutical composition for oral administration of [11] or [12], wherein the content of the additive which allows water to penetrate into the modified release portion is about 5% by weight to about 75% by weight with respect to the weight of the modified release portion,
[14] the pharmaceutical composition for oral administration of any one of [1] to [13], which is a pharmaceutical composition for treating urinary urgency, urinary frequency, and/or urge urinary incontinence associated with overactive bladder,
[15] the pharmaceutical composition for oral administration of any one of [1] to [14], wherein the pharmaceutical composition is a tablet,
[16] the pharmaceutical composition for oral administration of [15], which is a bi-layered tablet, and
[17] a pharmaceutical composition for oral administration comprising (1) a layer comprising mirabegron or a pharmaceutically acceptable salt thereof, and (2) a layer comprising solifenacin or a pharmaceutically acceptable salt thereof, and calcium stearate.

### ADVANTAGEOUS EFFECTS OF INVENTION

The present invention provides a pharmaceutical composition for oral administration comprising a modified release portion containing mirabegron or a pharmaceutically acceptable salt thereof, and an immediate release portion containing solifenacin or a pharmaceutically acceptable salt thereof. The pharmaceutical composition for oral administration of the present invention has a drug release similar to that of each current formulation (single drug formulation), and thus, a single formulation (combined formulation) capable of expecting pharmacological effects equivalent to those of the single drug formulations can be provided. Further, in providing a single formulation (combined formulation), a formulation capable of avoiding failures in tableting, such as lamination and sticking, and coloration of the immediate release portion during storage, can be provided. Furthermore, it is expected to improve drug dosing compliance, because the number of formulations to be administered is decreased.

### DESCRIPTION OF EMBODIMENTS

Embodiments of the present invention will be explained hereinafter.

The term "single drug formulation" as used herein means an embodiment of a formulation containing a drug.

The term "single formulation" as used herein is also referred to as "combined formulation", and means an embodiment of a formulation containing two or more drugs in the formulation. The term "single formulation" includes a formulation containing subformulations functionally different in releasing properties, such as the modified release portion and the immediate release portion in the present invention. As an embodiment of the "single formulation", multi-layered tablets, such as a bi-layered tablet in which the modified release portion and the immediate release portion are laminated, a multi-layered tablet in which a plurality of the modified release portion(s) and the immediate release portion(s) are laminated, and a three-layered tablet in which a drug-free layer is sandwiched between the modified release portion and the immediate release portion; a dry-coated tablet having the modified release portion as an internal core and the immediate release portion as an outer layer; and a film-coated tablet in which the modified release portion as a core is coated with the immediate release portion by film coating, may be exemplified. As another embodiment, a bi-layered tablet may be exemplified.

The term "modified release portion" as used herein means a portion which is contained in the single formulation, and which controls the release of the drug.

The term "immediate release portion" as used herein means a portion which is contained in the single formulation, and which rapidly releases the drug from the pharmaceutical composition (in the case of a "soluble" drug, "release" has almost the same meaning as "dissolution"). The term "rapidly release (dissolve)" or "rapid release (dissolution)" means that the release of the drug is not controlled. More particularly, it is defined by the dissolution rate of solifenacin, as described below, and it means, for example, that when a dissolution test is carried out in accordance with the Dissolution Test, method 1 (basket method, 100 rpm) described in the Japanese Pharmacopoeia, 85% or more of the drug is dissolved after 15 minutes.

The term "maximum percentage of dissolution" as used herein means the percentage of dissolution when a dissolution rate of the drug from the pharmaceutical composition reaches a plateau in a dissolution test under predetermined conditions.

The pharmaceutical composition for oral administration of the present invention will be explained hereinafter.

Mirabegron or a pharmaceutically acceptable salt thereof to be used in the present invention, is easily available by preparing it in accordance with, for example, the method described in Patent literature 2, or a modified method thereof.

Mirabegron may be used in a free form which is not a salt, and may form pharmaceutically acceptable salts with acids. Examples of the salts include acid addition salts with mineral acids such as hydrochloric acid, hydrobromic acid, hydriodic acid, sulfuric acid, nitric acid, and phosphoric acid; and acid addition salts with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, and glutamic acid. These salts can be prepared by a conventional method.

The dose of mirabegron in the single formulation (combined formulation) may be appropriately determined in accordance with the symptoms, the age and the sex of a patient to be treated, and the like. When mirabegron is orally administered to an adult, the daily dose is 0.01 mg/kg to 100 mg/kg, and is administered once or divided into two to four doses per day.

The content of mirabegron per modified release portion is, for example, 1% by weight to 70% by weight, 5% by weight to 70% by weight as another embodiment, and 5% by weight to 50% by weight as still another embodiment. The content of mirabegron per formulation is 1 mg to 500 mg, and 10 mg to 200 mg as another embodiment.

Solifenacin or a pharmaceutically acceptable salt thereof to be used in the present invention, is easily available by preparing it in accordance with the method described in Patent literature 5, or a modified method thereof.

Solifenacin may be used in a free form which is not a salt, and may form pharmaceutically acceptable salts with acids. Examples of the salts include acid addition salts with mineral acids such as hydrochloric acid, hydrobromic acid, hydriodic acid, sulfuric acid, nitric acid, and phosphoric acid; and acid addition salts with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, and glutamic acid. Solifenacin succinate may be exemplified in another embodiment. These salts can be prepared by a conventional method.

The dose of solifenacin in the single formulation (combined formulation) may be appropriately determined for each patient in accordance with, for example, the route of administration, symptoms of a disease, the age and the sex of a patient to be treated, and the like. When solifenacin succinate is orally administered to an adult, the daily dose is approximately 0.01 mg/kg to 100 mg/kg, and is administered once or divided into two to four doses per day.

The content of solifenacin is not particularly limited, so long as it is an effective amount for treatment or prevention. The content of solifenacin with respect to the immediate release portion is, for example, 0.5% by weight to 85% by weight, 0.5% by weight to 80% by weight as another embodiment, 0.5% by weight to 50% by weight as still another embodiment, and 0.5% by weight to 10% by weight as still another embodiment. The content of solifenacin per formulation is 0.01 mg to 100 mg as an embodiment, 0.5 mg to 50 mg as another embodiment, 0.5 mg to 20 mg as still another embodiment, and 0.5 mg to 10 mg as still another embodiment.

The "modified release portion" in the present invention is a formulation in which the drug release rate after 30 minutes from the beginning of a dissolution test is less than 85%, and is a formulation capable of controlling the release of a drug to the extent that the effects of food intake are decreased. The dissolution test may be carried out, for example, in accordance with the Dissolution Test (paddle method) described in the United States Pharmacopeia, using 900 mL of an appropriate test fluid (for example, a USP phosphate buffer (pH 6.8)), at a paddle rotation speed of 100 rpm, or in accordance with the Dissolution Test, method 2 described in the Japanese Pharmacopoeia, using 900 mL of an appropriate test fluid (for example, a Mc. Ilvain buffer (pH 6.8)), at a paddle rotation speed of 50 rpm to 200 rpm. More particularly, it is a formulation prepared by combining a hydrogel-forming polymer with an additive which allows water to penetrate into the formulation (hydrophilic base).

The hydrogel-forming polymer to be used in the present invention, is not particularly limited, so long as it can control the drug releasing rate, to the extent that the blood concentration profile of the drug is not affected by the presence or absence of food intake.

The molecular weight of the hydrogel-forming polymer is, for example, 100,000 or more, 100,000 to 8,000,000 in another embodiment, 100,000 to 5,000,000 in still another embodiment, and 100,000 to 2,000,000 in still another embodiment. The viscosity of the hydrogel-forming polymer is, for example, 12 mPa·s or more in a 5% aqueous solution at 25°C; 12 mPa·s or more in a 5% aqueous solution at 25°C, and 40,000 mPa·s or less in a 1% aqueous solution at 25°C in another embodiment; 400 mPa·s or more in a 2% aqueous solution at 25°C, and 7,500 mPa·s or less in a 1% aqueous solution at 25°C in still another embodiment; and 400 mPa·s or more in a 2% aqueous solution at 25°C, and 5,500 mPa·s or less in a 1% aqueous solution at 25°C in still another embodiment.

Examples of the hydrogel-forming polymer to be used in the present invention, include polyethylene oxide, hypromellose, hydroxypropylcellulose, methylcellulose, carboxymethylcellulose sodium, hydroxyethylcellulose, and carboxyvinyl polymers. Examples of the hydrogel-forming polymer in another embodiment include polyethylene oxide, hypromellose, and hydroxypropylcellulose. Examples of the hydrogel-forming polymer in still another embodiment include polyethylene oxide.

Examples of polyethylene oxide (hereinafter sometimes referred to as PEO) include product names, Polyox WSR-308 [average molecular weight: 8,000,000, viscosity: 10,000-15,000 mPa·s (1% aqueous solution at 25°C)], Polyox WSR-303 [average molecular weight: 7,000,000, viscosity: 7,500-10,000 mPa·s (1% aqueous solution at 25°C)], Polyox WSR Coagulant [average molecular weight: 5,000,000, viscosity: 5,500-7,500 mPa·s (1% aqueous solution at 25°C)], Polyox WSR-301 [average molecular weight: 4,000,000, viscosity: 1,650-5,500 mPa·s (1% aqueous solution at 25°C)], Polyox WSR-N-60K [average molecular weight: 2,000,000, viscosity: 2,000-4,000 mPa·s (2% aqueous solution at 25°C)], Polyox WSR-N-12K [average molecular weight: 1,000,000, viscosity: 400-800 mPa·s (2% aqueous solution at 25°C)], Polyox WSR-1105 [average molecular weight: 900,000, viscosity: 8,800-17,600 mPa·s (5% aqueous solution at 25°C)], Polyox WSR-205 [average molecular weight: 600,000, viscosity: 4,500-8,800 mPa·s (5% aqueous solution at 25°C)], Polyox WSR-N-750 [average molecular weight: 300,000, viscosity: 600-1200 mPa·s (5% aqueous solution at 25°C)], Polyox WSR-N-80 [average molecular weight: 200,000, viscosity: 55-90 mPa·s (5% aqueous solution at 25°C)], and Polyox WSR-N-10 [average molecular weight: 100,000, viscosity: 12-50 mPa·s (5% aqueous solution at 25°C)](manufactured by DOW).

Examples of hypromellose (hereinafter sometimes referred to as HPMC) include product name Metolose 90SH50000 [viscosity in a 2% aqueous solution at 20°C: 2,900-3,900 mPa·s], Metolose SB-4 (product name, Shin-Etsu Chemical Co., Ltd.)(viscosity in a 2% aqueous solution at 20°C: approximately 4 mPa·s), TC-5RW (product name, Shin-Etsu Chemical Co., Ltd.)(viscosity in a 2% aqueous solution at 20°C: approximately 6 mPa·s), TC-5S (product name, Shin-Etsu Chemical Co., Ltd.)(viscosity in a 2% aqueous solution at 20°C: approximately 15 mPa·s), TC-5R (product name, Shin-Etsu Chemical Co., Ltd.)(viscosity in a 2% aqueous solution at 20°C: approximately 6 mPa·s), TC-5M (product name, Shin-Etsu Chemical Co., Ltd.)(viscosity in a 2% aqueous solution at 20°C: approximately 4.5 mPa·s), TC-5E (product name, Shin-Etsu Chemical Co., Ltd.)(viscosity in a 2% aqueous solution at 20°C: approximately 3 mPa·s), Metolose 60SH-50 (product name, Shin-Etsu Chemical Co., Ltd.)(viscosity in a 2% aqueous solution at 20°C: approximately 50 mPa·s), Metolose 65SH-50 (product name, Shin-Etsu Chemical Co., Ltd.)(viscosity in a 2% aqueous solution at 20°C: approximately 50 mPa·s), Metolose 90SH-100 (product name, Shin-Etsu Chemical Co., Ltd.)(viscosity in a 2% aqueous solution at 20°C: approximately 100 mPa·s), Metolose 90SH-100SR (product name, Shin-Etsu Chemical Co., Ltd.)(viscosity in a 2% aqueous solution at 20°C: approximately 100 mPa·s), Metolose 65SH-400 (product name, Shin-Etsu Chemical Co., Ltd.)(viscosity in a 2% aqueous solution at 20°C: approximately 400 mPa·s), Metolose 90SH-400 (product name, Shin-Etsu Chemical Co., Ltd.)(viscosity in a 2% aqueous solution at 20°C: approximately 400 mPa·s), Metolose 65SH-1500 (product name, Shin-Etsu Chemical Co., Ltd.)(viscosity in a 2% aqueous solution at 20°C: approximately 1,500 mPa·s), Metolose 60SH-4000 (product name, Shin-Etsu Chemical Co., Ltd.)(viscosity in a 2% aqueous solution at 20°C: approximately 4,000 mPa·s), Metolose 65SH-4000 (product name, Shin-Etsu Chemical Co., Ltd.)(viscosity in a 2% aqueous solution at 20°C: approximately 4,000 mPa·s), Metolose 90SH-4000 (product name, Shin-Etsu Chemical Co., Ltd.)(viscosity in a 2% aqueous solution at 20°C: approximately 4,000 mPa·s), Metolose 90SH-4000SR (product name, Shin-Etsu Chemical Co., Ltd.)(viscosity in a 2% aqueous solution at 20°C: approximately 4,000 mPa·s), Metolose 90SH-15000 (product name, Shin-Etsu Chemical Co., Ltd.)(viscosity in a 2% aqueous solution at 20°C: approximately 15,000 mPa·s), Metolose 90SH-15000SR (product name, Shin-Etsu Chemical Co., Ltd.)(viscosity in a 2% aqueous solution at 20°C: approximately 15,000 mPa·s), and Metolose 90SH-30000 (product name, Shin-Etsu Chemical Co., Ltd.)(viscosity in a 2% aqueous solution at 20°C: approximately 30,000 mPa·s).

Examples of hydroxypropylcellulose (hereinafter sometimes referred to as HPC) include HPC-SSL (product name, Nippon Soda Co., Ltd.)(viscosity in a 2% aqueous solution at 20°C: 2.0-2.9 mPa·s), HPC-SL (product name, Nippon Soda Co., Ltd.)(viscosity in a 2% aqueous solution at 20°C: 3.0-5.9 mPa·s), HPC-L (product name, Nippon Soda Co., Ltd.)(viscosity in a 2% aqueous solution at 20°C: 6.0-10.0 mPa·s), HPC-M (product name, Nippon Soda Co., Ltd.)(viscosity in a 2% aqueous solution at 20°C: 150-400 mPa·s), and HPC-H (product name, Nippon Soda Co., Ltd.)(viscosity in a 2% aqueous solution at 20°C: 1,000-4,000 mPa·s).

Examples of methylcellulose (hereinafter sometimes referred to as MC) include Metolose SM15 (product name, Shin-Etsu Chemical Co., Ltd.)(viscosity in a 2% aqueous solution at 20°C: approximately 15 mPa·s), Metolose SM25 (product name, Shin-Etsu Chemical Co., Ltd.)(viscosity in a 2% aqueous solution at 20°C: approximately 25 mPa·s), Metolose SM100 (product name, Shin-Etsu Chemical Co., Ltd.)(viscosity in a 2% aqueous solution at 20°C: approximately 100 mPa·s), Metolose SM400 (product name, Shin-Etsu Chemical Co., Ltd.)(viscosity in a 2% aqueous solution at 20°C: approximately 400 mPa·s), Metolose SM1500 (product name, Shin-Etsu Chemical Co., Ltd.)(viscosity in a 2% aqueous solution at 20°C: approximately 1,500 mPa·s), and Metolose SM4000 (product name, Shin-Etsu Chemical Co., Ltd.)(viscosity in a 2% aqueous solution at 20°C: approximately 4,000 mPa·s).

Examples of carboxymethylcellulose sodium (hereinafter sometimes referred to as CMCNa) include product names, Sunrose F-30MC [viscosity: 250-350 mPa·s (1% aqueous solution at 25°C)], Sunrose F-150MC [average molecular weight: 200,000, viscosity: 1,200-1,800 mPa·s (1% aqueous solution at 25°C)], Sunrose F-600MC [viscosity: 6,000-8,000 mPa·s (1% aqueous solution at 25°C)], Sunrose F-1000MC [average molecular weight: 420,000, viscosity: 8,000-12,000 mPa·s (1% aqueous solution at 25°C)], Sunrose F-1400MC [viscosity: 12,000-15,000 mPa·s (1% aqueous solution at 25°C)], and Sunrose F-300MC [average molecular weight: 300,000, viscosity: 2,500-3,000 mPa·s (1% aqueous solution at 25°C)](manufactured by Nippon Paper Chemicals Co., Ltd.).

Examples of hydroxyethylcellulose (hereinafter sometimes referred to as HEC) include product names, HEC DAICEL SE850 [average molecular weight: 1,480,000, viscosity: 2,400-3,000 mPa·s (1% aqueous solution at 25°C)], and HEC DAICEL SE900 [average molecular weight: 1,560,000, viscosity: 4,000-5,000 mPa·s (1% aqueous solution at 25°C)] (manufactured by Daicel chemical Industries, Ltd.).

Examples of carboxyvinyl polymers include Carbopol 940 (average molecular weight: approximately 2,500,000, manufactured by B.F.Goodrich Chemical).

These hydrogel-forming polymers may be used alone, or as an appropriate combination of two or more thereof. A combination of different lots may be used.

The content of the hydrogel-forming polymer is not particularly limited, so long as it is an amount to the extent that the blood concentration profile of the drug is not affected by the presence or absence of food intake. The content of the hydrogel-forming polymer with respect to the modified release portion is, for example, 1% by weight to 70% by weight, 3% by weight to 70% by weight as another embodiment, 5% by weight to 70% by weight as still another embodiment, 10% by weight to 60% by weight as still another embodiment, and 10% by weight to 40% by weight as still another embodiment. The content of the hydrogel-forming polymer per formulation is 1% by weight to 45% by weight, 2% by weight to 45% by weight as another embodiment, 3% by weight to 45% by weight as still another embodiment, 5% by weight to 35% by weight as still another embodiment, and 5% by weight to 25% by weight as still another embodiment. The content of the hydrogel-forming gel with respect to the weight of the drug is 0.1% by weight to 1000% by weight, 1% by weight to 500% by weight as another embodiment, and 5% by weight to 300% by weight as still another embodiment.

A polymer of which the viscosity (before mixing) is beyond the specific range can be used as an appropriate combination with one or more other polymers, in cases where the mixture obtained by mixing these plural polymers has a viscosity (as measured before use) within the specific range.

In the additive which allows water to penetrate into the modified release portion (hydrophilic base) to be used in the present invention, the amount of water necessary to dissolve 1 g of the hydrophilic base at 20±5°C is 10 mL or less, 6 mL or less in another embodiment, 5 mL or less in still another embodiment, and 4 mL or less in still another embodiment. When the hydrophilic base has a higher solubility to water, the effect that allows water to penetrate into the formulation is higher.

Examples of the hydrophilic base include: water-soluble polymers, such as polyethylene glycol [PEG: for example, product names PEG 400, PEG 1500, PEG 4000, PEG 6000, and PEG 20000 (manufactured by NOF Corporation)], polyvinylpyrrolidone [PVP: for example, product name PVP K30 (manufactured by BASF)], and the like; sugar alcohols, such as D-mannitol, D-sorbitol, xylitol, and the like; saccharides, such as lactose, sucrose, anhydrous maltose, D-fructose, dextran (for example, Dextran 40), glucose, and the like; surfactants, such as polyoxyethylene hydrogenated castor oil [HCO: for example, Cremophor RH40 (manufactured by BASF), HCO-40, HCO-60 (manufactured by Nikko Chemicals)], polyoxyethylene polyoxypropylene glycol [for example, Pluronic F68 (manufactured by Asahi Denka and the like)], polyoxyethylene sorbitan higher fatty acid esters [Tween: for example, Tween 80 (manufactured by Kanto Chemical)], and the like; salts, such as sodium chloride, magnesium chloride, and the like; organic acids, such as citric acid, tartaric acid, and the like; amino acids, such as glycine, β-alanine, lysine hydrochloride, and the like; and aminosaccharides, such as meglumine, and the like.

As another embodiment, PEG, PVP, D-mannitol, D-sorbitol, xylitol, lactose, sucrose, anhydrous maltose, D-fructose, dextran, glucose, polyoxyethylene polyoxypropylene glycol, sodium chloride, magnesium chloride, citric acid, tartaric acid, glycine, β-alanine, lysine hydrochloride, or meglumine may be exemplified. As still another embodiment, PEG, PVP, D-mannitol, lactose, sucrose, sodium chloride, or polyoxyethylene polyoxypropylene glycol may be exemplified. As still another embodiment, PEG may be exemplified.

These hydrophilic bases may be used alone, or as an appropriate combination of two or more thereof.

The content of the hydrophilic base is not particularly limited, so long as it is an amount capable of controlling the release of the drug to the extent that the release of the drug is not affected by food. The content of the hydrophilic base with respect to the modified release portion is, for example, 5% by weight to 75% by weight, 5% by weight to 70% by weight in another embodiment, and 20% by weight to 60% by weight in still another embodiment. The content of the hydrophilic base per formulation is 3% by weight to 44% by weight as an embodiment, 3% by weight to 40% by weight as another embodiment, and 12% by weight to 35% by weight as still another embodiment.

An antioxidant may be contained in the modified release portion in the present invention. The antioxidant is not particularly limited, so long as the influence of dissolution behavior can be avoided. Examples of the antioxidant include butylated hydroxytoluene (BHT), propyl gallate (PG), butylhydroxyanisol (BHA), ascorbic acid, sodium ascorbate, erythorbic acid, sodium nitrite, sodium bisulfite, sodium pyrosulfite, citric acid, and edetate sodium; BHT, PG, and sodium ascorbate in another embodiment; and BHT in still another embodiment. These antioxidants may be used alone, or as an appropriate combination of two or more thereof. The content of the antioxidant with respect to the modified release portion is, for example, 0.025% by weight to 0.25% by weight. As another embodiment, the content of the antioxidant per formulation is 0.015% by weight to 0.15% by weight.

A stabilizer may be contained in the modified release portion in the present invention. When polyethylene oxide is used as the hydrogel-forming polymer, the stabilizer is not particularly limited, so long as it does not periodically change the release properties of the drug. Examples of the stabilizer include yellow ferric oxide, red ferric oxide, black iron oxide, and the like. These stabilizers may be used alone, or as an appropriate combination of two or more thereof. The content of the stabilizer with respect to the weight of the modified release portion is 0.05% by weight to 1% by weight. As another embodiment, the content of the stabilizer with respect to the formulation is 0.03% by weight to 0.6% by weight.

The dissolution rate of solifenacin from the immediate release portion in the present invention is not particularly limited, so long as it shows availability in the living body equivalent to that of the current solifenacin formulation (single drug formulation). When a test is carried out in accordance with, for example, the Dissolution Test, method 2 described in the Japanese Pharmacopoeia (paddle method, 50 rpm to 200 rpm), the Dissolution Test, method 1 described in the Japanese Pharmacopoeia (basket method, 50 rpm to 200 rpm), the Dissolution Test (paddle method) described in the United States Pharmacopeia, the Dissolution Test (basket method) described in the United States Pharmacopeia, or the like, it is defined as follows: (1) after 15 minutes, 85% or more of a drug is dissolved, and 90% or more is dissolved in another embodiment, (2) after 30 minutes, 90% or more of a drug is dissolved, and 95% or more is dissolved in another embodiment, and (3) after 60 minutes, 90% or more of a drug is dissolved, 95% or more is dissolved in another embodiment, and 97% or more is dissolved in still another embodiment. Another embodiment of the test method is the Dissolution Test, method 1 described in the Japanese Pharmacopoeia (basket method, 100 rpm). The dissolution rate of solifenacin from the immediate release portion is defined, from these dissolution rates (1), (2), and (3), alone, or as a combination of two or more.

The maximum percentage of solifenacin dissolution from the immediate release portion in the present invention is not particularly limited, so long as it shows availability in the living body equivalent to that of the current solifenacin formulation (single drug formulation). When a test is carried out in accordance with, for example, the Dissolution Test, method 2 described in the Japanese Pharmacopoeia (paddle method, 50 rpm to 200 rpm), the Dissolution Test, method 1 described in the Japanese Pharmacopoeia (basket method, 50 rpm to 200 rpm), the Dissolution Test (paddle method) described in the United States Pharmacopeia, the Dissolution Test (basket method) described in the United States Pharmacopeia, or the like, it is defined by a drug dissolution rate after 60 minutes. As another embodiment, when a test is carried out in accordance with the Dissolution Test, method 1 described in the Japanese Pharmacopoeia (basket method, 100 rpm), it is defined by a drug dissolution rate after 60 minutes. The maximum percentage of solifenacin dissolution from the immediate release portion is defined as that the drug dissolution rate after 60 minutes is 90% or more, that the drug dissolution rate after 60 minutes is 92% or more in another embodiment, that the drug dissolution rate after 60 minutes is 95% or more in still another embodiment, and that the drug dissolution rate after 60 minutes is 97% or more in still another embodiment.

A filler and/or a binder contained in the immediate release portion to be used in the present invention are not limited, so long as they are pharmaceutically acceptable, and pharmacologically acceptable. Examples of the filler and/or the binder include lactose, D-mannitol, maltose, polyethylene glycol, polyvinyl pyrrolidone, hypromellose, and hydroxypropylcellulose. As another embodiment, D-mannitol, maltose, polyethylene glycol, and polyvinyl pyrrolidone are exemplified. As still another embodiment, D-mannitol and maltose, and D-mannitol and hydroxypropylcellulose are exemplified. Examples of the polyethylene glycol include, for example, PEG 400, PEG 1500, PEG 4000, PEG 6000, and PEG 20000 (Product names, manufactured by NOF Corporation)]. Examples of the polyvinyl pyrrolidone include, for example, Kollidon K25 and Kollidon K90 (Product names, manufactured by BASF), and the like.

The content of the filler and/or the binder contained in the immediate release portion, with respect to the weight of the immediate release portion, is, for example, 5% by weight to 99% by weight, 40% by weight to 99% by weight in another embodiment, 80% by weight to 99% by weight in still another embodiment, and 90% by weight to 99% by weight in still another embodiment. The content of the filler and/or the binder contained in the immediate release portion, with respect to the weight of the formulation, is 5% by weight to 50% by weight as an embodiment, 10% by weight to 40% by weight as another embodiment, 20% by weight to 40% by weight as still another embodiment, and 30% by weight to 40% by weight as still another embodiment.

These fillers and/or binders may be used alone, or as an appropriate combination of two or more thereof.

Calcium stearate contained in the immediate release portion to be used in the present invention is not particularly limited, so long as it conforms to the standards of the Japanese Pharmacopoeia, the United States Pharmacopeia, the European Pharmacopoeia, or the like. For example, Parteck (trademark) LUB CST (product name, manufactured by MERCK) may be exemplified. The content of calcium stearate with respect to the weight of the immediate release portion is, for example, 0.1% by weight to 10% by weight, 0.5% by weight to 3.0% by weight as another embodiment, 0.5% by weight to 2.0% by weight as still another embodiment, and 0.5% by weight to 1.5% by weight as still another embodiment. The content of calcium stearate with respect to the weight of the formulation is 0.05% by weight to 6% by weight, and 0.2% by weight to 1% by weight as another embodiment.

Various pharmaceutical additives may be appropriately used to prepare the pharmaceutical composition for oral administration of the present invention, if desired, and are not particularly limited, so long as they are pharmaceutically and pharmacologically acceptable. Examples of the pharmaceutical additive include a filler, a binder, a disintegrating agent, an acidulant, an effervescent agent, an artificial sweetener, a flavor, a coloring agent, a buffer, an antioxidant, a surfactant, and the like.

Examples of the filler to be used in the modified release portion include lactose, sucrose, D-mannitol, D-sorbitol, starch, gelatinized starch, dextrin, crystalline cellulose, low substituted hydroxypropylcellulose, carboxymethylcellulose sodium, gum arabic, dextrin, pullulan, light anhydrous silicic acid, synthetic aluminum silicate, magnesium aluminate metasilicate, and the like.

Examples of the binder to be used in the modified release portion include gum arabic, hypromellose, hydroxypropylcellulose, hydroxyethylcellulose, and the like.

Examples of the disintegrating agent include corn starch, potato starch, carmellose calcium, carmellose sodium, low-substituted hydroxypropylcellulose, and the like.

Examples of the acidulant include citric acid, tartaric acid, malic acid, and the like.

Examples of the effervescent agent include sodium bicarbonate, and the like.

Examples of the artificial sweetener include saccharin sodium, dipotassium glycyrrhizinate, aspartame, stevia, thaumatin, and the like.

Examples of the flavor include lemon, lemon-lime, orange, menthol, and the like.

Examples of the coloring agent include food yellow No. 4, food yellow No. 5, food red No. 3, food red No. 102, food blue No. 3, and the like.

Examples of the buffer include citric acid, succinic acid, fumaric acid, tartaric acid, ascorbic acid, and salts thereof; glutamic acid, glutamine, glycine, aspartic acid, alanine, arginine, and salts thereof; and magnesium oxide, zinc oxide, magnesium hydroxide, phosphoric acid, boric acid, and salts thereof.

Examples of the antioxidant include ascorbic acid, dibutyl hydroxytoluene, propyl gallate, and the like.

Examples of the surfactant include polysorbate 80, sodium laurylsulfate, polyoxyethylene hydrogenated castor oil, and the like.

These pharmaceutical additives may be appropriately added alone, or as a combination of two or more thereof, in an appropriate amount.

With respect to the contents, each pharmaceutical additive may be contained in an amount such that the desired effects of the present invention may be achieved.

As the pharmaceutical composition (formulation) for oral administration of the present invention, a single formulation (combined formulation) prepared by a known method per se, such as a pharmaceutical composition for oral administration containing a layer comprising mirabegron or a pharmaceutically acceptable salt thereof and a layer comprising solifenacin or a pharmaceutically acceptable salt thereof, may be exemplified. As another embodiment, multi-layered tablets, such as a bi-layered tablet in which a layer comprising mirabegron or a pharmaceutically acceptable salt thereof and a layer comprising solifenacin or a pharmaceutically acceptable salt thereof are laminated, a multi-layered tablet in which a plurality of a layer(s) comprising mirabegron or a pharmaceutically acceptable salt thereof and a layer(s) comprising solifenacin or a pharmaceutically acceptable salt thereof are laminated, and a three-layered tablet in which a drug-free layer is sandwiched between a layer comprising mirabegron or a pharmaceutically acceptable salt thereof and a layer comprising solifenacin or a pharmaceutically acceptable salt thereof; a dry-coated tablet having a modified release portion comprising mirabegron or a pharmaceutically acceptable salt thereof as an internal core and an immediate release portion comprising solifenacin or a pharmaceutically acceptable salt thereof as an outer layer; and a film-coated tablet in which a modified release portion comprising mirabegron or a pharmaceutically acceptable salt thereof as a core is coated with an immediate release portion comprising solifenacin or a pharmaceutically acceptable salt thereof by film coating, may be exemplified. As still another embodiment, a bi-layered tablet in which a layer comprising mirabegron or a pharmaceutically acceptable salt thereof and a layer comprising solifenacin or a pharmaceutically acceptable salt thereof are laminated, may be exemplified.

In the layer comprising mirabegron or a pharmaceutically acceptable salt thereof, a substance or two or more substances selected from the group consisting of a hydrogel-forming polymer, a hydrophilic base, an antioxidant, a stabilizer, and a pharmaceutical additive may be contained. As these substances, the above-mentioned substances may be used.

In the layer comprising solifenacin or a pharmaceutically acceptable salt thereof, a substance or two or more substances selected from the group consisting of a filler, a binder, calcium stearate, and a pharmaceutical additive may be contained. As these substances, the above-mentioned substances may be used.

Hereinafter the process of manufacturing the pharmaceutical composition for oral administration of the present invention will be explained in detail.

The pharmaceutical composition for oral administration of the present invention may be produced by appropriately combining known methods per se.

### (1) Pulverizing and mixing steps

An apparatus and a method used in the pulverizing step are not particularly limited, so long as drugs and appropriate pharmaceutical additives can be pharmaceutically pulverized. Examples of the apparatus include a hammer mill, a ball mill, a jet mill, a colloid mill, and the like. The conditions for pulverization may be appropriately selected and are not particularly limited.

An apparatus and a method used in the mixing step subsequent to the pulverizing step are not particularly limited, so long as components can be uniformly mixed pharmaceutically.

### (2) Modified release portion: granulation step

An apparatus and a method used in this step are not particularly limited, so long as the hydrogel-forming polymer and the like can be granulated.

Examples of a granulation method and a granulation apparatus include a high-speed agitation granulation method, a pulverization granulation method, a fluidized bed granulation method, an extrusion granulation method, a tumbling granulation method, and a spray granulation method; and apparatuses used in these methods. A fluidized bed granulation method and apparatus may be used in another embodiment, and a tumbling fluidized bed granulation method and apparatus may be used in still another embodiment. The resulting granulated product may be dried. The drying method is not particularly limited, so long as the granulated product can be pharmaceutically dried.

### (3) Immediate release portion: granulation step

An apparatus and a method used in this step are not particularly limited, so long as the drugs and the like can be granulated.

Examples of a producing method and a producing apparatus include a fluidized bed granulation method, a melting granulation, a high-speed agitation granulation method, a pulverization granulation method, an extrusion granulation method, a tumbling granulation method, a spray granulation method, and a dry granulation method; and apparatuses used in these methods. A fluidized bed granulation method and apparatus may be used in another embodiment.

Binders used in wet granulation may be used alone, or as an appropriate combination of two or more thereof.

In the spray granulation method, the resulting granulated product may be dried. The drying method is not particularly limited, so long as the granulated product can be pharmaceutically dried.

### (4) Forming step

An apparatus and a method used in this step are not particularly limited, so long as the pharmaceutical composition for oral administration of the present invention can be formed. Examples of the method include: a method in which the drugs and appropriate pharmaceutical additives are mixed without granulation and drying, and directly compression-molded to obtain tablets; a method in which the granulation step is carried out, a lubricant is added to the resulting granulated product, and the mixture is compression-molded to obtain tablets; a method of preparing bi-layered tablets by laminating the modified release portion and the immediate release portion; a method of preparing multi-layered tablets by laminating a plurality of the modified release portion(s) and the immediate release portion(s);
a method of preparing multi-layered tablets by adding a drug-free layer between the modified release portion and the immediate release portion; and
a method of preparing dry-coated tablets having the modified release portion as an internal core and the immediate release portion as an outer layer. As another embodiment, a method of preparing bi-layered tablets may be exemplified.

Examples of a tableting machine include a multi-layered rotary tableting machine, an oil press, and the like.

The conditions for tableting, such as a tableting pressure, are not particularly limited, so long as bi-layered tablets and/or multi-layered tablets can be prepared. When bi-layered tablets are prepared, a granulated product for the first layer and another granulated product for the second layer are laminated, and compressed under a tableting pressure of approximately 2 kN to approximately 20 kN to prepare the bi-layered tablets. In another embodiment, a granulated product for the first layer is compressed under a tableting pressure of approximately 0.1 kN to approximately 10 kN, and another granulated product for the second layer is placed on the first layer and compressed under a tableting pressure of approximately 2 kN to approximately 20 kN to prepare the bi-layered tablets. When multi-layered tablets are prepared, a tableting pressure can be appropriately adjusted to carry out the compression.

The hardness of the resulting tablet is not particularly limited, so long as the tablet is not damaged during the manufacturing process, the distribution process, and the like. The hardness may be, for example, 40 N to 200 N.

### (5) Film coating

After tableting, the obtained tablets may be film coated.

The method of film coating is not particularly limited, so long as the tablets can be pharmaceutically coated. Examples of the coating include pan coating, dip coating, and the like.

Film coating agents may be added alone, or as a combination of two or more thereof, in an appropriate amount. The coating rate is not particularly limited, so long as a film can be formed. The coating rate is, for example, 1% to 10%.

When a core comprising the modified release portion is coated with the immediate release portion to prepare film coated tablets, a spray liquid prepared by dissolving and/or dispersing the components of the immediate release portion in a solvent such as water may be sprayed on the core to obtain the film coated tablets. The coating rate is not particularly limited, so long as the film comprising the immediate release portion can be formed. The coating rate is, for example, 1% to 20%, or the like.

After the film coating, the resulting film coated tablets may be dried. The drying method is not particularly limited, so long as the film coated tablets can be pharmaceutically dried. The conditions for drying are not particularly limited, so long as they are appropriately selected in view of, for example, the stability of the formulation. The initial water content after film coating is preferably 0.1% to 2% in accordance with, for example, the stability.

The pharmaceutical composition for oral administration of the present invention may be used as a pharmaceutical composition for treating urinary urgency, urinary frequency, and/or urge urinary incontinence associated with overactive bladder.

The process of manufacturing the pharmaceutical composition for oral administration of the present invention includes, in addition to the above-mentioned methods, methods of producing a pharmaceutical composition by appropriately combining known methods per se.

### EXAMPLES

The present invention will be further illustrated by, but is by no means limited to, the following Examples, Comparative Examples, Referential Examples, and Experimental Examples.

### Example 1

### (1) Preparation of mixed powder for modified release portion

After 6.0 parts of mirabegron was pulverized, using a screen mill (COMIL, manufactured by Powrex Corporation), together with 16.8 parts of polyethylene oxide (POLYOX (registered trademark) N-60K, manufactured by Dow, the same compound was used in the following Examples.), 34.7 parts of polyethylene glycol 8000 (Polyglykol 8000PF, manufactured by Clariant, the same compound was used in the following Examples.), and 1.8 parts of hydroxypropylcellulose (HPC-SL, manufactured by Nippon Soda Co. Ltd., The same compound was used in the following Examples.), the resulting pulverized powder was loaded into a fluidized bed granulating apparatus (GPCG-120, manufactured by Powrex Corporation), and granulated by spraying 6.7 parts of water. With 59.3 parts of the dried granulated product, 0.1 parts of butylhydroxytoluene (dibutylhydroxytoluene, manufactured by MERCK/EMD, the same compound was used in the following Examples.) and 0.6 parts of magnesium stearate (Parteck (registered trademark) LUB MST, manufactured by MERCK, the same compound was used in the following Examples.) were mixed to obtain mixed powder for a modified release portion.

### (2) Preparation of mixed powder for immediate release portion

A spray liquid was prepared by dissolving 4.0 parts of maltose (Sunmalt S, manufactured by Sanwa Starch Co., Ltd., The same compound was used in the following Examples.) in 16.0 parts of water while stirring. Into a fluidized bed granulating apparatus (GPCG-1, manufactured by Powrex), 0.6 parts of solifenacin succinate was loaded, together with 35 parts of mannitol (Pearitol 50C, manufactured by Roquette, The same compound was used in the following Examples.), and granulated by spraying the spray liquid. With the 39.6 parts of the dried granulated product, 0.4 parts of calcium stearate was mixed to obtain mixed powder for an immediate release portion.

### (3) Tableting

Using an oil press tableting machine (Autograph AGS-20KNG, manufactured by Shimadzu Corporation, the same apparatus was used in the following Examples.), 60 parts of the mixed powder for a modified release portion and 40 parts of the mixed powder for an immediate release portion were formed into bi-layered tablets, to obtain a pharmaceutical composition (bi-layered tablets) for oral administration of the present invention containing 25 mg of mirabegron and 2.5 mg of solifenacin succinate. As a Referential Example, the mixed powder for an immediate release portion was formed into tablets to obtain a single drug formulation consisting of an immediate release portion.

### Example 2

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 1.2 parts of hydroxypropylcellulose in 10.8 parts of water while stirring. Into a fluidized bed granulating apparatus (FLO-01, manufactured by Freund Corporation), 0.6 parts of solifenacin succinate was loaded, together with 37.8 parts of mannitol, and granulated by spraying the spray liquid. With the 39.6 parts of the dried granulated product, 0.4 parts of calcium stearate was mixed to obtain mixed powder for an immediate release portion.

### (3) Tableting

Using an oil press tableting machine, 60 parts of the mixed powder for a modified release portion and 40 parts of the mixed powder for an immediate release portion were formed into bi-layered tablets, to obtain a pharmaceutical composition (bi-layered tablets) for oral administration of the present invention containing 25 mg of mirabegron and 2.5 mg of solifenacin succinate. As a Referential Example, the mixed powder for an immediate release portion was formed into tablets to obtain a single drug formulation consisting of an immediate release portion.

### Example 3

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.1 parts of water while stirring. Into a fluidized bed granulating apparatus (FLO-01, manufactured by Freund Corporation), 1.2 parts of solifenacin succinate was loaded, together with 34.5 parts of mannitol, and granulated by spraying the spray liquid. With the 39.7 parts of the dried granulated product, 0.2 parts of calcium stearate was mixed to obtain mixed powder for an immediate release portion.

### (3) Tableting

Using an oil press tableting machine, 60.1 parts of the mixed powder for a modified release portion and 39.9 parts of the mixed powder for an immediate release portion were formed into bi-layered tablets, to obtain a pharmaceutical composition (bi-layered tablets) for oral administration of the present invention containing 25 mg of mirabegron and 5 mg of solifenacin succinate.

### Example 4

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.0 parts of water while stirring. Into a fluidized bed granulating apparatus (GPCG-15, manufactured by Powrex), 1.2 parts of solifenacin succinate was loaded, together with 34.4 parts of mannitol, and granulated by spraying the spray liquid. With the 39.6 parts of the dried granulated product, 0.4 parts of calcium stearate was mixed to obtain mixed powder for an immediate release portion.

### (3) Tableting

Using a tri-layered tableting machine(HT-CVX45LS-UW/3Lvtt, manufactured by Hata Iron Works Co., Ltd.), 60 parts of the mixed powder for a modified release portion and 40 parts of the mixed powder for an immediate release portion were formed into bi-layered tablets, to obtain a pharmaceutical composition (bi-layered tablets) for oral administration of the present invention containing 25 mg of mirabegron and 5 mg of solifenacin succinate. As a Referential Example, the mixed powder for an immediate release portion was formed into tablets, using an oil press tableting machine, to obtain a single drug formulation consisting of an immediate release portion.

### Example 5

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.0 parts of water while stirring. Into a fluidized bed granulating apparatus (FLO-01, manufactured by Freund Corporation), 1.2 parts of solifenacin succinate was loaded, together with 34.3 parts of mannitol, and granulated by spraying the spray liquid. With the 39.5 parts of the dried granulated product, 0.6 parts of calcium stearate was mixed to obtain mixed powder for an immediate release portion.

### (3) Tableting

Using an oil press tableting machine, 59.9 parts of the mixed powder for a modified release portion and 40.1 parts of the mixed powder for an immediate release portion were formed into bi-layered tablets, to obtain a pharmaceutical composition (bi-layered tablets) for oral administration of the present invention containing 25 mg of mirabegron and 5 mg of solifenacin succinate.

### Example 6

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.0 parts of water while stirring. Into a fluidized bed granulating apparatus (FLO-01, manufactured by Freund Corporation), 1.2 parts of solifenacin succinate was loaded, together with 34.2 parts of mannitol, and granulated by spraying the spray liquid. With the 39.4 parts of the dried granulated product, 0.8 parts of calcium stearate was mixed to obtain mixed powder for an immediate release portion.

### (3) Tableting

Using an oil press tableting machine, 59.8 parts of the mixed powder for a modified release portion and 40.2 parts of the mixed powder for an immediate release portion were formed into bi-layered tablets, to obtain a pharmaceutical composition (bi-layered tablets) for oral administration of the present invention containing 25 mg of mirabegron and 5 mg of solifenacin succinate.

### Example 7

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 15.9 parts of water while stirring. Into a fluidized bed granulating apparatus (FLO-01, manufactured by Freund Corporation), 1.2 parts of solifenacin succinate was loaded, together with 34.1 parts of mannitol, and granulated by spraying the spray liquid. With the 39.3 parts of the dried granulated product, 1.2 parts of calcium stearate was mixed to obtain mixed powder for an immediate release portion.

### (3) Tableting

Using an oil press tableting machine, 59.5 parts of the mixed powder for a modified release portion and 40.5 parts of the mixed powder for an immediate release portion were formed into bi-layered tablets, to obtain a pharmaceutical composition (bi-layered tablets) for oral administration of the present invention containing 25 mg of mirabegron and 5 mg of solifenacin succinate.

### Comparative Example 1

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.0 parts of water while stirring. Into a fluidized bed granulating apparatus (GPCG-1, manufactured by Powrex), 0.6 parts of solifenacin succinate was loaded, together with 35 parts of mannitol, and granulated by spraying the spray liquid. With the 39.6 parts of the dried granulated product, 0.4 parts of magnesium stearate was mixed to obtain mixed powder for an immediate release portion.

### (3) Tableting

Using an oil press tableting machine, 60 parts of the mixed powder for a modified release portion and 40 parts of the mixed powder for an immediate release portion were formed into bi-layered tablets, to obtain a pharmaceutical composition (bi-layered tablets) for oral administration of the present invention containing 25 mg of mirabegron and 2.5 mg of solifenacin succinate. As a Referential Example, the mixed powder for an immediate release portion was formed into tablets to obtain a single drug formulation consisting of an immediate release portion.

### Comparative Example 2

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.0 parts of water while stirring. Into a fluidized bed granulating apparatus (GPCG-1, manufactured by Powrex), 0.6 parts of solifenacin succinate was loaded, together with 35 parts of mannitol, and granulated by spraying the spray liquid. With the 39.6 parts of the dried granulated product, 0.4 parts of sodium stearyl fumarate (PRUV (registered trademark), manufactured by Rettenmaier Japan Co., Ltd.) was mixed to obtain mixed powder for an immediate release portion.

### (3) Tableting

Using an oil press tableting machine, 60 parts of the mixed powder for a modified release portion and 40 parts of the mixed powder for an immediate release portion were formed into bi-layered tablets, to obtain a pharmaceutical composition (bi-layered tablets) for oral administration for comparison containing 25 mg of mirabegron and 2.5 mg of solifenacin succinate.

### Comparative Example 3

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.0 parts of water while stirring. Into a fluidized bed granulating apparatus (FLO-01, manufactured by Freund Corporation), 1.2 parts of solifenacin succinate was loaded, together with 34.4 parts of mannitol, and granulated by spraying the spray liquid. With the 39.6 parts of the dried granulated product, 0.4 parts of magnesium stearate was mixed to obtain mixed powder for an immediate release portion.

### (3) Tableting

Using an oil press tableting machine, 60 parts of the mixed powder for a modified release portion and 40 parts of the mixed powder for an immediate release portion were formed into bi-layered tablets, to obtain a pharmaceutical composition (bi-layered tablets) for oral administration for comparison containing 25 mg of mirabegron and 5 mg of solifenacin succinate. As a Referential Example, the mixed powder for an immediate release portion was formed into tablets to obtain a single drug formulation consisting of an immediate release portion.

### Comparative Example 4

### (1) Preparation of mixed powder for modified release portion

A modified release portion was obtained under the same formulation and production conditions as those described in Example 1.

### (2) Preparation of mixed powder for immediate release portion

A spray liquid was prepared by dissolving 4.0 parts of maltose in 16.0 parts of water while stirring. Into a fluidized bed granulating apparatus (FLO-01, manufactured by Freund Corporation), 1.2 parts of solifenacin succinate was loaded, together with 34.4 parts of mannitol, and granulated by spraying the spray liquid. With the 39.6 parts of the dried granulated product, 0.4 parts of sodium stearate (sodium stearate, manufactured by Nacalai Tesque, Inc.) was mixed to obtain mixed powder for an immediate release portion.

### (3) Tableting

Using an oil press tableting machine, 60 parts of the mixed powder for a modified release portion and 40 parts of the mixed powder for an immediate release portion were formed into bi-layered tablets, to obtain a pharmaceutical composition (bi-layered tablets) for oral administration for comparison containing 25 mg of mirabegron and 5 mg of solifenacin succinate. As a Referential Example, the mixed powder for an immediate release portion was formed into tablets to obtain a single drug formulation consisting of an immediate release portion.

### Experimental Example 1

The pharmaceutical compositions (bi-layered tablets) for oral administration prepared in Examples 1 and 2, and Comparative Examples 1 and 2 were subjected to a dissolution test carried out in accordance with the Dissolution Test, method 1 (basket method, 100 rpm) described in the Japanese Pharmacopoeia. As a test fluid, 900 mL of water was used. The dissolution rates of solifenacin after 15 minutes, 30 minutes, and 60 minutes from the beginning of the test were evaluated by an HPLC method. The results of the dissolution test for solifenacin are shown in Table 1. The dissolution rate of solifenacin and the maximum percentage of solifenacin dissolution from the pharmaceutical compositions (bi-layered tablets) for oral administration prepared in Comparative Examples 1 and 2 were lower than those of the pharmaceutical compositions (bi-layered tablets) for oral administration prepared in Examples 1 and 2.

**Table 1**

| | 15 min. | 30 min. | 60 min. |
|---|---|---|---|
| Ex. 1 | 92.5% | 96.5% | 97.5% |
| Ex. 2 | 93.7% | 97.5% | 98.8% |
| Comp. Ex. 1 | 82.0% | 86.0% | 88.0% |
| Comp. Ex. 2 | 82.2% | 84.5% | 85.7% |

### Experimental Example 2

The pharmaceutical compositions (bi-layered tablets) for oral administration prepared in Examples 3 to 7, and Comparative Examples 3 and 4 were subjected to a dissolution test carried out in accordance with the Dissolution Test, method 1 (basket method, 100 rpm) described in the Japanese Pharmacopoeia. As a test fluid, 900 mL of a USP phosphate buffer (pH 6.8) was used. The dissolution rates of solifenacin after 15 minutes, 30 minutes, and 60 minutes from the beginning of the test were evaluated by an HPLC method. The results of the dissolution test for solifenacin are shown in Table 2. The dissolution rate of solifenacin and the maximum percentage of solifenacin dissolution from the pharmaceutical compositions (bi-layered tablets) for oral administration prepared in Comparative Examples 3 and 4 were lower than those of the pharmaceutical compositions (bi-layered tablets) for oral administration prepared in Examples 3 to 7.

**Table 2**

| | 15 min. | 30 min. | 60 min. |
|---|---|---|---|
| Ex. 3 | 93.5% | 94.8% | 96.2% |
| Ex. 4 | 97.8% | 99.3% | 101.0% |
| Ex. 5 | 91.2% | 92.7% | 94.3% |
| Ex. 6 | 93.5% | 95.5% | 96.2% |
| Ex. 7 | 92.3% | 94.7%, | 95.7% |
| Comp. Ex. 3 | 82.8% | 86.8% | 90.7% |
| Comp. Ex. 4 | 75.5% | 78.5% | 82.7% |

### Experimental Example 3

With respect to the pharmaceutical compositions (bi-layered tablets) for oral administration prepared in Example 1 and Comparative Example 1, productivity in tableting (presence or absence of sticking), and lamination, which is a problem specific to laminated tablets, were evaluated by visual observation. The results of the evaluation are shown in Table 3. In the pharmaceutical composition prepared in Example 1, the failures in tableting, i.e., sticking and lamination, were not observed.

**Table 3**

| | Sticking | Incident of lamination (25°C, 60% RII, 30 days) |
|---|---|---|
| Ex. 1 | Not occur | 0 % (0/20 samples) |
| Comp. Ex. 1 | Occur | 80 % (16/20 samples) |

### Experimental Example 4

With respect to the pharmaceutical compositions (bi-layered tablets) for oral administration prepared in Example 1 and Comparative Example 1, a change in appearance (coloration of the immediate release portion) during storage was periodically evaluated by visual observation. The results of the evaluation are shown in Table 4. In the pharmaceutical composition prepared in Example 1, the coloration of the immediate release portion was not observed.

**Table 4**

| | 60°C 21 days | 50°C 30 days | 40°C, 75% RH 30 days |
|---|---|---|---|
| Ex. 1 | Coloration: Not observed (0/10 samples) | Coloration: Not observed (0/10 samples) | Coloration: Not observed (0/10 samples) |
| Comp, Ex. 1 | Coloration: Observed (10/10 samples) | Coloration: Observed (10/10 samples) | Coloration: Not observed (0/10 samples) |

### Experimental Example 5

With respect to the immediate release portions (single drug formulations) of the pharmaceutical compositions for oral administration prepared, as Reference Examples, in Examples 1, 2, and 4 and Comparative Examples 1, 3, and 4, a disintegration time thereof was measured in accordance with the Disintegration Test, described in the Japanese Pharmacopoeia, in order to confirm the disintegration time. The results of the measurement are shown in Table 5. All the immediate release portions (single drug formulations) of the pharmaceutical compositions for oral administration prepared in Examples 1, 2, and 4 and Comparative Examples 1, 3, and 4 disintegrated within 300 seconds.

**Table 5**

| | Ex. 1 | Ex. 2 | Ex. 4 | Comp. Ex. 1 | Comp. Ex. 3 | Comp. Ex. 4 |
|---|---|---|---|---|---|---|
| Disintegration Time | 43 sec. | 66 sec. | 254 sec. | 33 sec. | 279 sec. | 248 sec. |

### Experimental Example 6

The immediate release portions (single drug formulations) of the pharmaceutical compositions for oral administration prepared, as Reference Examples, in Examples 1 and 2 and Comparative Example 1 were subjected to a dissolution test carried out in accordance with the Dissolution Test, method 1 (basket method, 100 rpm) described in the Japanese Pharmacopoeia. As a test fluid, 900 mL of water was used. The dissolution rates of solifenacin after 15 minutes, 30 minutes, and 60 minutes from the beginning of the test were evaluated by a UV method. The results of the dissolution test for solifenacin are shown in Table 6. The immediate release portions (single drug formulations) of the pharmaceutical compositions for oral administration prepared in Examples 1 and 2 and Comparative Example 1 showed similar dissolution rates of solifenacin and similar maximum percentages of solifenacin dissolution.

**Table 6**

| | 15 min. | 30 min. | 60 min. |
|---|---|---|---|
| Ex. 1 | 97.3% | 98.7% | 98.0% |
| Ex.2 | 99.3% | 100.7% | 101.5% |
| Comp. Ex. 1 | 94.6% | 100.7% | 100.0% |

### Experimental Example 7

The immediate release portions (single drug formulations) of the pharmaceutical compositions for oral administration prepared, as Reference Examples, in Example 4 and Comparative Examples 3 and 4 were subjected to a dissolution test carried out in accordance with the Dissolution Test, method 1 (basket method, 100 rpm) described in the Japanese Pharmacopoeia. As a test fluid, 900 mL of a USP phosphate buffer (pH 6.8) was used. The dissolution rates of solifenacin after 15 minutes, 30 minutes, and 60 minutes from the beginning of the test were evaluated by a UV method. The results of the dissolution test for solifenacin are shown in Table 7. The immediate release portions (single drug formulations) of the pharmaceutical compositions for oral administration prepared in Example 4 and Comparative Examples 3 and 4 showed similar dissolution rates of solifenacin and similar maximum percentages of solifenacin dissolution.

**Table 7**

| | 15 min. | 30 min. | 60 min. |
|---|---|---|---|
| Ex. 4 | 95.5% | 96.0% | 96.2% |
| Comp. Ex. 3 | 90.7% | 94.2% | 96.7% |
| Comp. Ex. 4 | 85.7% | 89.8% | 93.1% |

### INDUSTRIAL APPLICABILITY

The present invention provides a pharmaceutical composition for oral administration comprising a modified release portion containing mirabegron or a pharmaceutically acceptable salt thereof, and an immediate release portion containing solifenacin or a pharmaceutically acceptable salt thereof. The pharmaceutical composition for oral administration of the present invention exhibits a drug release rate similar to those of the current formulations (single drug formulations), and thus, can be used as a formulation technique which provides a single formulation (a combined formulation) capable of expecting pharmacological effects equivalent to those of the current formulations (single drug formulations).

Although the present invention has been described with reference to specific embodiments, various changes and modifications obvious to those skilled in the art are possible without departing from the scope of the appended claims.

## Claims

1. A pharmaceutical composition for oral administration comprising:
(1) a modified release portion comprising mirabegron or a pharmaceutically acceptable salt thereof, and
(2) an immediate release portion comprising solifenacin or a pharmaceutically acceptable salt thereof, and calcium stearate.

2. The pharmaceutical composition for oral administration according to claim 1, wherein the immediate release portion is disintegrated and/or dissolved before the modified release portion forms a gel.

3. The pharmaceutical composition for oral administration according to claim 1 or 2, wherein about 85% or more of solifenacin is dissolved after 15 minutes.

4. The pharmaceutical composition for oral administration according to claim 3, wherein 90% or more of solifenacin is dissolved after 60 minutes.

5. The pharmaceutical composition for oral administration according to any one of claims 1 to 4, wherein the content of calcium stearate is about 0.1% by weight to about 10% by weight with respect to the weight of the immediate release portion.

6. The pharmaceutical composition for oral administration according to any one of claims 1 to 5, wherein the modified release portion contains a polymer which forms a hydrogel.

7. The pharmaceutical composition for oral administration according to claim 6, wherein the hydrogel-forming polymer has an average molecular weight of about 100,000 or more, or a viscosity of 12 mPa·s or more in a 5% aqueous solution at 25°C.

8. The pharmaceutical composition for oral administration according to claim 7, wherein the hydrogel-forming polymer is one polymer or two or more polymers selected from the group consisting of polyethylene oxide, hypromellose, hydroxypropylcellulose, methylcellulose, carboxymethylcellulose sodium, hydroxyethylcellulose, and a carboxyvinyl polymer.

9. The pharmaceutical composition for oral administration according to claim 8, wherein the hydrogel-forming polymer is polyethylene oxide.

10. The pharmaceutical composition for oral administration according to any one of claims 6 to 9, wherein the content of the hydrogel-forming polymer is about 1% by weight to about 70% by weight with respect to the weight of the modified release portion.

11. The pharmaceutical composition for oral administration according to any one of claims 1 to 10, wherein the modified release portion further contains an additive which allows water to penetrate into the modified release portion.

12. The pharmaceutical composition for oral administration according to claim 11, wherein the additive which allows water to penetrate into the modified release portion has a solubility such that the amount of water necessary to dissolve 1 g of the additive is 10 mL or less.

13. The pharmaceutical composition for oral administration according to claim 11 or 12, wherein the content of the additive which allows water to penetrate into the modified release portion is about 5% by weight to about 75% by weight with respect to the weight of the modified release portion.

14. The pharmaceutical composition for oral administration according to any one of claims 1 to 13, which is a pharmaceutical composition for treating urinary urgency, urinary frequency, and/or urge urinary incontinence associated with overactive bladder.

15. The pharmaceutical composition for oral administration according to any one of claims 1 to 14, wherein the pharmaceutical composition is a tablet.

16. The pharmaceutical composition for oral administration according to claim 15, which is a bi-layered tablet.

17. A pharmaceutical composition for oral administration comprising:
(1) a layer comprising mirabegron or a pharmaceutically acceptable salt thereof, and
(2) a layer comprising solifenacin or a pharmaceutically acceptable salt thereof, and calcium stearate.
